# EUROPEAN PATENT SPECIFICATION

(11) **EP 1 929 953 B1**
(45) Date of publication and mention of the grant of the patent: **27.06.2012**
(21) Application number: 06745715.0
(22) Date of filing: 26.04.2006
(51) Int. Cl.: A61B 8/06

(54) **ULTRASONOGRAPH**
ULTRASCHALLGERÄT
ULTRASONOGRAPHE

(30) Priority: 26.09.2005 JP 2005278749
(43) Date of publication of application: 11.06.2008
(73) Proprietor: OLYMPUS MEDICAL SYSTEMS CORP., Tokyo 151-0072 (JP)
(72) Inventor: SABATA, Tomohiro c/o OLYMPUS MEDICAL SYSTEMS CORP.,, Tokyo 151-0072 (JP); OBA, Kenichi c/o OLYMPUS MEDICAL SYSTEMS CORP.,, Tokyo 151-0072 (JP); KANBARA, Tadaaki c/o OLYMPUS MEDICAL SYSTEMS CORP.,, Tokyo 151-0072 (JP)
(74) Representative: Katérle, Axel
(86) International application number: PCT/JP2006/308737
(87) International publication number: WO 2007/034590

(56) References cited:
- JP-A- 08 164 138
- JP-A- 10 075 949
- JP-A- 10 286 256
- JP-A- 11 276 481

## Description

### Technical Field

The present invention relates to an ultrasound diagnostic apparatus, particularly to an ultrasound diagnostic apparatus having a function of showing dynamic blood flow in a living body.

### Background Art

Conventionally, there has been widely used an ultrasound diagnostic apparatus which irradiates ultrasound into a living body and receives a reflected wave of the ultrasound reflected at a tissue of the living body to obtain a tomogram of the living body. Further, in recent years, there has been also widely used an ultrasound diagnostic apparatus which has a function of displaying, besides the tomogram of a living body, for example, dynamic blood flow in a living body as a blood flow image by exploiting the Doppler effect by which the frequency of a reflected wave is shifted due to the moving velocity of a reflecting object in motion.

As such an apparatus, for example, a blood-flow velocity display apparatus proposed in Japanese Patent Publication No. 06-9556 is widely known. The blood-flow velocity display apparatus of Japanese Patent Publication No. 06-9556 is configured such that after a comparison between the magnitudes of a blood-flow velocity calculation value during a current scan and a blood-flow velocity display value during the previous scan is performed by blood-flow velocity display value calculation means, a blood-flow velocity display value is calculated based on the aforementioned comparison result and is used to display a blood flow image on a color monitor. Thus; using the blood-flow velocity display apparatus of Japanese Patent Publication No. 06-9556 enables an operator or the like to easily recognize a peak value of a pulsating blood flow velocity.

However, since the blood-flow velocity display apparatus of Japanese Patent Publication No. 06-9556 performs the comparison by the blood-flow velocity display value calculation means and the calculation based on the comparison result to display a blood flow image, the processing load on the blood-flow velocity display value calculation means tends to increase, thus the frame rate of the blood flow image may be reduced. This will result in a problem that the blood-flow velocity display apparatus of Japanese Patent Publication No. 06-9556 may not be able to accurately display dynamic blood flow.

JP 11 276481, on which document the preambles of claims 1 and 2 are based, discloses a system in which image processing is applied to information related to a power of a blood flow and a luminance value is thereafter assigned to the image processed blood flow intensity information.

The present invention has been made in view of the above described problem and its object is to provide an ultrasound diagnostic apparatus which enables to display dynamic blood flow more accurately than ever without sacrificing the frame rate of blood flow image.

### Disclosure of Invention

### Means for Solving the Problem

An ultrasound diagnostic apparatus according to claims 1 and 2 is provided. The ultrasound diagnostic apparatus includes ultrasound transmitting/receiving means for transmitting an ultrasound pulse to generate ultrasound to be transmitted from ultrasound transducing means to a test subject, and receiving an echo signal outputted from the ultrasound transducing means based on the ultrasound reflected in the test subject; based on the echo signal, dynamic blood flow detection means for calculating a predetermined detection value based on dynamic blood flow in the test subject; luminance value conversion means for converting the predetermined detection value into a luminance value; luminance value replacement means retaining luminance value conversion information for converting one luminance value to another luminance value; and comparison means for comparing a first luminance value in one frame with a second luminance value in the frame immediately preceding the one frame, out of luminance values outputted from the luminance value conversion means, wherein the comparison means is adapted to output either the first luminance value or a third luminance value, which is not larger than the absolute value of the second luminance value, based on the comparison result and the luminance value replacement information.

The ultrasound diagnostic apparatus of a first aspect is the ultrasound diagnostic apparatus according to the first aspect, wherein the comparison means is adapted to output the first luminance value when obtaining a comparison result that the first luminance value is larger than the second luminance value, and to replace the second luminance value with the third luminance value based on the luminance value replacement information, thereby outputting the third luminance value when obtaining a comparison result that the first luminance value is not larger than the second luminance value.

The ultrasound diagnostic apparatus of a preferred embodiment is an ultrasound diagnostic apparatus according to the second aspect , wherein the comparison means is adapted to increase the attenuation of the third luminance value as the second luminance value increases when replacing the second luminance value with the third luminance value based on the luminance value replacement information.

The ultrasound diagnostic apparatus of a preferred embodiment is an ultrasound diagnostic apparatus, wherein the luminance value replacement information retained by the luminance value replacement means is configured to be a rewritable data file.

The ultrasound diagnostic apparatus of a preferred embodiment is an ultrasound diagnostic apparatus, wherein the luminance value replacement information retained by the luminance value replacement means is configured to be a rewritable data file.

The ultrasound diagnostic apparatus of a preferred embodiment is an ultrasound apparatus, wherein the data file is made up of a plurality of data files.

The ultrasound diagnostic apparatus of a preferred embodiment is an ultrasound apparatus, wherein the data file is made up of a plurality of data files.

The ultrasound diagnostic apparatus of a second aspect is an ultrasound diagnostic apparatus, wherein the comparison means is adapted to output the first luminance value when obtaining a comparison result that the first luminance value has a different sign from that of the second luminance value, or that the first luminance value has the same sign as that of the second luminance value and the absolute value of the first luminance value is larger than that of the second luminance value; and to replace the second luminance value with the third luminance value based on the luminance value replacement information and output the third luminance value, when obtaining a comparison result that the first luminance value has the same sign as that of the second luminance and the absolute value of the first luminance value is not larger than that of the second luminance value.

The ultrasound diagnostic apparatus of a preferred embodiment of an ultrasound diagnostic apparatus, wherein the comparison means is adapted to increase the attenuation of the third luminance value as the second luminance value increases when replacing the second luminance value with the third luminance value based on the luminance value replacement information.

The ultrasound diagnostic apparatus of a preferred embodiment of an ultrasound diagnostic apparatus, wherein the luminance value replacement information retained by the luminance value replacement means is configured to be a rewritable data file.

The ultrasound diagnostic apparatus of a preferred embodiment of an ultrasound diagnostic apparatus, wherein the luminance value replacement information retained by the luminance value replacement means is configured to be a rewritable data file.

The ultrasound diagnostic apparatus of a preferred embodiment of an ultrasound diagnostic apparatus, wherein the data file is made up of a plurality of data files.

The ultrasound diagnostic apparatus of a preferred embodiment of an ultrasound diagnostic apparatus, wherein the data file is made up of a plurality of data files.

The ultrasound diagnostic apparatus of a preferred embodiment of an ultrasound diagnostic apparatus, wherein the luminance value replacement information retained by the luminance value replacement means is configured to be a rewritable data file.

The ultrasound diagnostic apparatus of a preferred embodiment of an ultrasound diagnostic apparatus wherein the data file is made up of a plurality of data files.

The ultrasound diagnostic apparatus of a preferred embodiment of an ultrasound diagnostic apparatus, wherein the comparison means is adapted to increase the attenuation of the third luminance value as the second luminance value increases when replacing the second luminance value with the third luminance value based on the luminance value replacement information.

The ultrasound diagnostic apparatus of a preferred embodiment of an ultrasound diagnostic apparatus, wherein the luminance value replacement information retained by the luminance value replacement means is configured to be a rewritable data file.

The ultrasound diagnostic apparatus of an embodiment of the ultrasound diagnostic apparatus, wherein the luminance value replacement information retained by the luminance value replacement means is configured to be a rewritable data file.

The ultrasound diagnostic apparatus of an embodiment of the ultrasound diagnostic apparatus, wherein the data file is made up of a plurality of data files.

The ultrasound diagnostic apparatus of an embodiment of the ultrasound diagnostic apparatus according to the nineteenth aspect , wherein the data file is made up of a plurality of data files.

The ultrasound diagnostic apparatus of an embodiment of the may include: ultrasound transmitting/receiving means for transmitting an ultrasound pulse to generate ultrasound to be transmitted from ultrasound transducing means to a test subject, and receiving an echo signal outputted from the ultrasound transducing means based on the ultrasound reflected in the test subject; based on the echo signal, dynamic blood flow detection means for calculating a predetermined detection value based on dynamic blood flow in the test subject; luminance value conversion means for converting the predetermined detection value into a luminance value; and comparison means for comparing a first luminance value in one frame with a second luminance value in the frame immediately preceding the one frame, out of the luminance values outputted from the luminance value conversion means, wherein the ultrasound diagnostic apparatus further includes a lookup table including a comparative table for converting the second luminance value into a third luminance value which is not larger than the absolute value of the second luminance value, and wherein the comparison means outputs either the first luminance value or the third luminance value based on the comparison result and the comparative table.

The ultrasound diagnostic apparatus of an embodiment of the ultrasound diagnostic apparatus according to the twenty-second aspect , wherein the comparative table retained by the lookup table is configured to be a rewritable data file.

The ultrasound diagnostic apparatus of a preferred embodiment of the ultrasound diagnostic apparatus, wherein the data file is made up of a plurality of data files.

### Brief Description of the Drawings

- Fig. 1: shows the configuration of principal parts of an ultrasound diagnostic system in which the ultrasound diagnostic apparatus relating to a present embodiment is used;
- Fig. 2: shows a configuration example of an after image processing circuit included in the ultrasound diagnostic apparatus of Fig. 1;
- Fig. 3: is a graph to show the relationship between the input luminance value and the output luminance value corresponding to the input luminance value in a comparative table included in the lookup table in the after image processing circuit of Fig. 2;
- Fig. 4: is a graph to show the variation of the luminance value of the portion where blood flow exists, in a blood flow image displayed on a monitor;
- Fig. 5: shows a configuration example of a variation of the after image processing circuit of Fig. 2; and
- Fig. 6: shows another example, which is different from that of Fig. 5, of a variation of the after image processing circuit of Fig. 2.

### Best Mode for Carrying Out the Invention

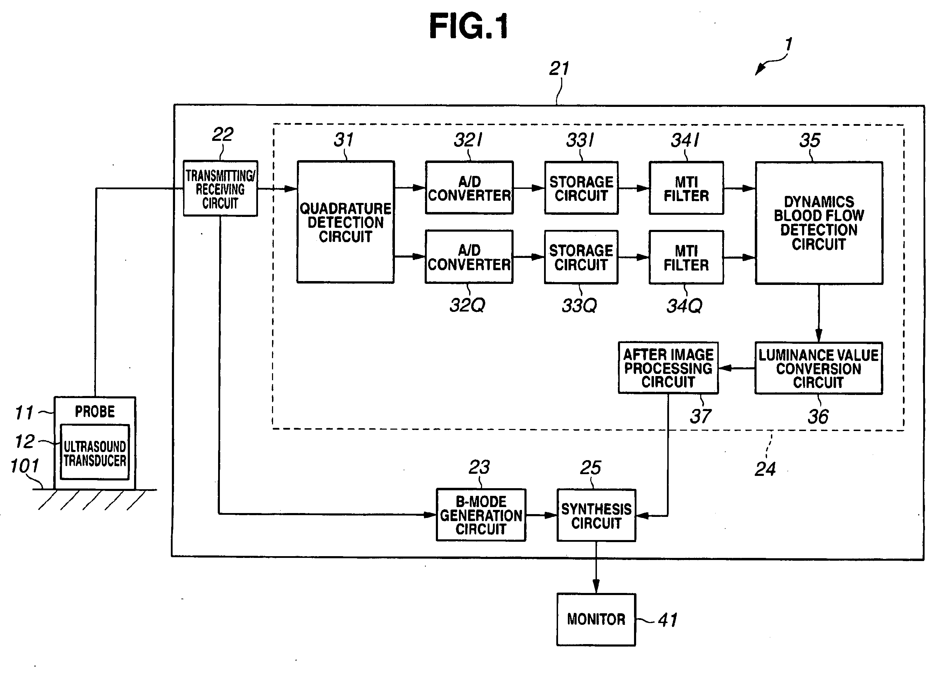

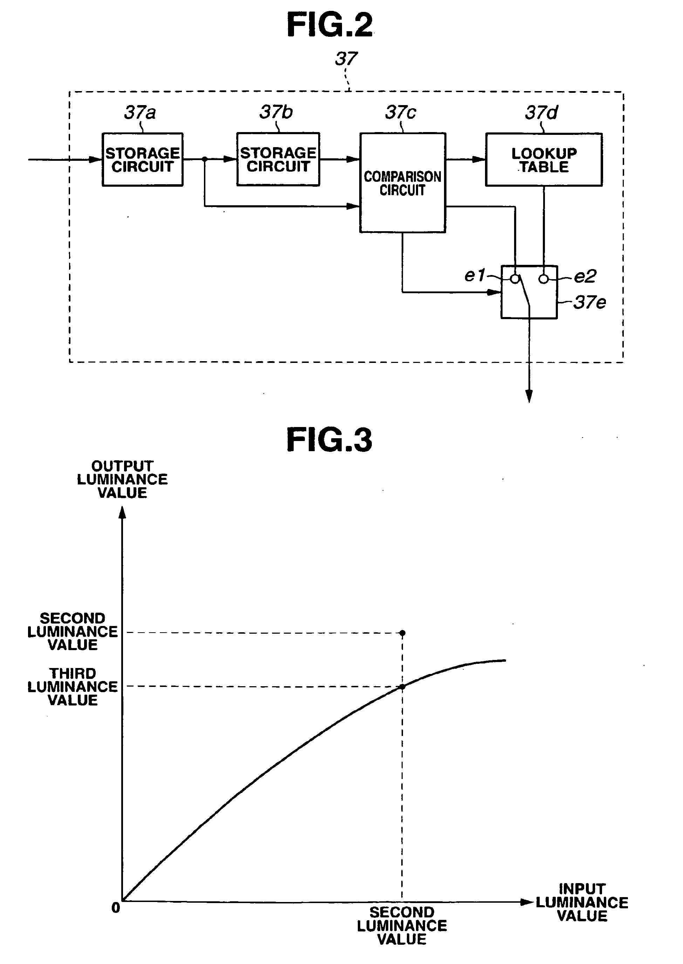

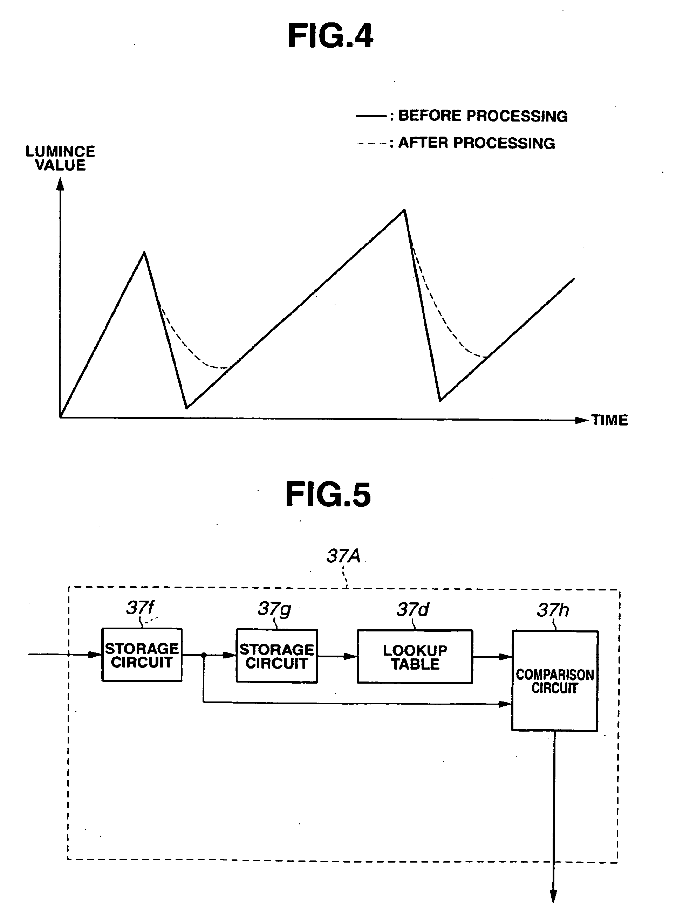

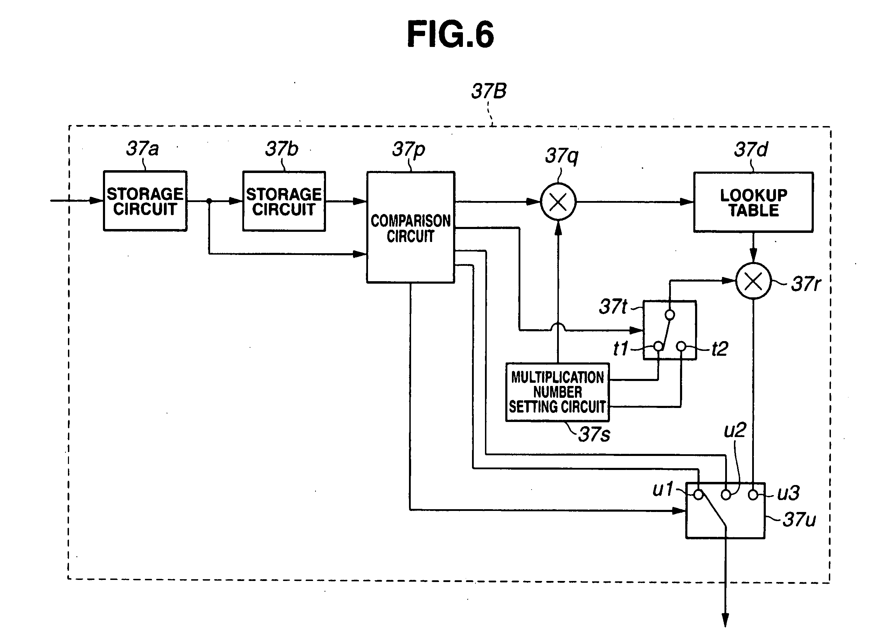

Hereinafter, embodiments of the present invention will be described with reference to the drawings. Fig. 1 shows the configuration of principal parts of an ultrasound diagnostic system in which the ultrasound diagnostic apparatus relating to a present embodiment is used. Fig. 2 shows a configuration example of an after image processing circuit included in the ultrasound diagnostic apparatus of Fig. 1. Fig. 3 is a graph to show the relationship between the input luminance value and the output luminance value corresponding to the input luminance value in a comparative table included in the lookup table in the after image processing circuit of Fig. 2. Fig. 4 is a graph to show the variation of the luminance value of the portion where blood flow exists, in a blood flow image displayed on a monitor. Fig. 5 shows a configuration example of a variation of the after image processing circuit of Fig. 2. Fig. 6 shows another example, which is different from that of Fig. 5, of a variation of the after image processing circuit of Fig. 2.

The ultrasound diagnostic system 1 is configured to include a probe 11, an ultrasound diagnostic apparatus 21, and a monitor 41.

The probe 11 contains thereinside an ultrasound transducer 12 which transmits ultrasound to a test subject 101, for example, a living body, and receives a reflected wave of the ultrasound in the test subject 101.

The ultrasound transducer 12 as the ultrasound transducing means transmits ultrasound to the test subject 101 based on the ultrasound pulse signal from the ultrasound diagnostic apparatus 21, and receives the reflected wave of the ultrasound in the test subject 101 to output it as an echo signal to the ultrasound diagnostic apparatus 21.

Further, the ultrasound diagnostic apparatus 21 is configured to include a transmitting/receiving circuit 22, a B-mode generation circuit 23, a Doppler processing circuit 24, and a synthesis circuit 25.

The transmitting/receiving circuit 22 as ultrasound transmitting/receiving means transmits an ultrasound pulse signal for driving the ultrasound transducer 12 to the probe 11 and receives the echo signal outputted from the ultrasound transducer 12 to perform processing thereon amplification and the like, and outputs the processed echo signal to the B-mode generation circuit 23 and the Doppler processing circuit 24.

The B-mode generation circuit 23 generates a tomogram of the test subject 101 based on the echo signal outputted from the transmitting/receiving circuit 22 and thereafter outputs the tomogram as a tomography signal to the synthesis circuit 25.

The Doppler processing circuit 24 is configured to include a quadrature detection circuit 31, A/D (analog/digital) converters 32I and 32Q, storage circuits 33I and 33Q, MTI (Moving Target Indicator) filters 34I and 34Q, a dynamic blood flow detection circuit 35, a luminance value conversion circuit 36, and an after image processing circuit 37.

The quadrature detection circuit 31 performs quadrature detection based on the echo signal outputted from the transmitting/receiving circuit 22 to output an in-phase echo signal, which is an in-phase component of the echo signal, to the A/D converter 32I and also to output a quadrature echo signal, which is a quadrature component of the echo signal, to the A/D converter 32Q.

The A/D converter 32I digitally converts an in-phase echo signal outputted from the quadrature detection circuit 31 and outputs the digitally converted in-phase echo signal to the storage circuit 331. Further, the A/D converter 32Q digitally converts a quadrature echo signal outputted from the quadrature detection circuit 31 and outputs the digitally converted quadrature echo signal to the storage circuit 33Q.

Further, configuration may be such that the echo signal outputted from the transmitting/receiving circuit 22 is converted into a digital signal by the A/D converter and thereafter is subjected to quadrature detection by a quadrature detection circuit made up of a digital filter.

The storage circuit 33I made up of a memory and the like stores, for example, only one frame of the in-phase echo signal outputted from the A/D converter 32I as the signal value which will be needed for the processing performed by the dynamic blood flow detection circuit 35, and thereafter outputs the signal value of the in-phase echo signal to the MTI filter 34I. Further, the storage circuit 33Q made up of a memory and the like stores, for example, only one frame of the quadrature echo signal outputted from the A/D converter 32Q as the signal value which will be needed for the processing performed by the dynamic blood flow detection circuit 35, and thereafter outputs the signal value of the in-phase echo signal to the MTI filter 34Q.

The MTI filter 341 performs a processing to remove a component, which moves slowly in a living body like an organ, from the in-phase echo signal outputted from the storage circuit 33I and thereafter outputs the processed in-phase echo signal to the dynamic blood flow detection circuit 35. Further, MTI filter 34Q performs a processing to remove a component, which moves slowly in a living body like an organ, from the quadrature echo signal outputted from the storage circuit 33Q and thereafter outputs the processed quadrature echo signal to the dynamic blood flow detection circuit 35.

The dynamic blood flow detection circuit 35 is configured to include: a complex autocorrelation processing circuit for generating and outputting complex data representing a complex autocorrelation vector based on the in-phase echo signal and the quadrature echo signal outputted from the MTI filters 341 and 34Q; a frame memory retaining a frame of complex data; a weighted addition circuit for weighting and adding the inputted complex data; a velocity/intensity calculation circuit for calculating the velocity and the intensity of blood flow, and the amplitude of the complex data, based on the weighted and added data; and a threshold processing circuit for removing a component with a velocity not larger than a predetermined velocity and a component with am amplitude not larger than a predetermined amplitude. With the above described configuration, the dynamic blood flow detection circuit 35 can perform the processing to calculate a velocity value to indicate the velocity of blood flow in a living body or a power value to indicate the intensity of blood flow in a living body, as the predetermined detection value based on the dynamic blood flow in a living body, based on the in-phase echo signal and quadrature echo signal outputted from the MTI filters 34I and 34Q. Further, the dynamic blood flow detection circuit 35 outputs the velocity value or the power value which has been obtained through the aforementioned processing, as velocity value data or power value data to the luminance value conversion circuit 36.

The luminance value conversion circuit 36 performs processing based on the Doppler effect on the velocity value data or the power value data outputted from the dynamic blood flow detection circuit 35 to convert the velocity value data into luminance value data for each frame, and thereafter outputs the luminance value data to the after image processing circuit 37.

The after image processing circuit 37 is configured, as shown in Fig. 2, to include storage circuits 37a and 37b, a comparison circuit 37c, a lookup table 37d, and a switch 37e.

The storage circuit 37a, which is made up of a frame memory and the like, temporarily stores first luminance value data, which is luminance value data in one frame, and second luminance value data, which is luminance value data in the frame immediately preceding the one frame, out of the luminance value data outputted from the luminance value conversion circuit 36, thereafter outputting the first luminance value data to the comparison circuit 37c as well as outputting the second luminance value data to the storage circuit 37b.

The storage circuit 37b, which is made up of a frame memory and the like, temporarily stores the second luminance value data outputted from the storage circuit 37a and thereafter outputs the second luminance value data to the comparison circuit 37c.

The comparison circuit 37c compares the first luminance value based on the first luminance value data outputted from the storage circuit 37a with the second luminance value based on the second luminance value data outputted from the storage circuit 37b. Moreover, the first luminance value and the second luminance value are both supposed to be a positive whole number not less than 0, for example, 0 to 255.

The comparison circuit 37c outputs the first luminance value data to the switch 37e when, for example, the first luminance value is larger than the second luminance value. Further, the comparison circuit 37c controls the switching of the switch 37e so as to turn a terminal e1 into a conduction state so that the first luminance value data is outputted to the synthesis circuit 25.

Further, for example, when the first luminance value is not larger than the second luminance value, the comparison circuit 37c performs the processing to replace the second luminance value data having the second luminance value with third luminance value data having a third luminance value described later making reference to the lookup table 37d, and thereafter outputs the third luminance value data to the switch 37e. Further, the comparison circuit 37c controls the switching of the switch 37e so as to turn a terminal e2 into a conduction state such that the third luminance value data is outputted to the synthesis circuit 25. Moreover, the third luminance value is supposed to be a positive whole number not less than 0, for example, 0 to 255.

The lookup table 37d as luminance value replacement means is configured to include a memory not shown, an input/output circuit, and the like. The memory not shown includes a comparative table containing luminous value conversion information which is to be referred to when the processing to replace a second luminance value based on the second luminance value data with a third luminance value which is preset as a value not larger than the absolute value of the second luminance value is performed. Further, the comparative table included in the lookup table 37d contains a plurality of combinations of an input luminance value, which is for example a whole number from 0 to 255, and an output luminance value, which is a whole number from 0 to 255, as the luminance value corresponding to the input luminance value. Further, each of the combinations of two luminance values has a relationship that the attenuation of the third luminance value, as the output luminance value, increases as the second luminance value, as the input luminance value, increases as shown as a nonlinear graph in Fig. 3.

In the present embodiment, the comparative table included in a memory not shown of the lookup table 37d is configured such that an input luminance value and the output luminance value corresponding to the input luminance value can be rewritten into a desired value, and for example can be configured to be a data file of a text data form. Further, the aforementioned data file is for example configured such that file identification data such as an identification code is written in the head of each data file, and in the rear of the portion in which the file identification data is written, for example, there are written a combination of two luminance values: an input luminance value which is a whole number from 0 to 255, and an output luminance value which corresponds to the input luminance value and is a whole number from 0 to 255.

Further, the lookup table 37d is not limited to a configuration in which the memory not shown has only one comparative table, and may be configured such that the memory not shown has a plurality of comparative tables. Further, when the memory not shown of the lookup table 37d has a plurality of comparative tables, the configuration may be such that an operator or the like can select a desired comparative table out of the plurality of comparative tables or that the comparison circuit 37c can select one comparative table depending on the comparison result.

The synthesis circuit 25 generates a blood flow image to show the dynamic state of blood flow in the test subject 101 based on the luminance value data outputted from the Doppler processing circuit 24. Then, the synthesis circuit 25 synthesizes a blood flow image generated based on the luminance value data outputted from the Doppler processing circuit 24 and a tomogram of the test subject 101 based on the tomography signal outputted from the B-mode generation circuit 23, and outputs the synthesized blood flow image and the tomogram as synthesized image data to the monitor 41.

The monitor 41 displays an image in which a tomogram of the test subject 101 as a living body is superimposed with a blood flow image based on the synthesized image signal outputted from the synthesis circuit 25.

Next, effects of the ultrasound diagnostic system 1 in the present embodiment will be described.

First, an operator or the like puts the probe 11 into contact with a desired observation site of the test subject 101 and thereafter makes the ultrasound transducer 12 oscillate by operating a switch not shown and provided on the exterior surface of the ultrasound diagnostic apparatus 21 to transmit ultrasound to the test subject 101.

After transmitting ultrasound to the test subject 101, the ultrasound transducer 12 receives the reflected wave of the ultrasound in the test subject 101 and outputs it as an echo signal to the ultrasound diagnostic apparatus 21.

The ultrasound diagnostic apparatus 21 generates a tomography signal at the B-mode generation circuit 23 based on the echo signal outputted from the ultrasound transducer 12 of the probe 11, and outputs either one of the first luminance value data or the third luminance value data at the Doppler processing circuit 24. The ultrasound diagnostic apparatus 21 synthesizes a blood flow image generated based on the luminance value data outputted from the Doppler processing circuit 24 and a tomogram of the test subject 101 based on the tomography signal outputted from the B-mode generation circuit 23, and outputs the blood flow image and the tomogram after synthesis as synthesized image data to the monitor 41.

The monitor 41 displays an image in which a tomogram of the test subject 101 as a living body is superimposed with a blood flow image to show dynamic blood flow in the test subject 101, based on the synthetic image signal outputted from the synthesis circuit 25.

With the above described series of processing being performed by the ultrasound diagnostic apparatus 21, an operator or the like can visually recognize dynamic blood flow in the test subject 101 in the blood flow image displayed on the monitor 41. Further, the blood flow image to be displayed on the monitor 41 may be shown for example as an image in which, when velocity value data is outputted from the dynamic blood flow detection circuit 35, the larger the blood flow velocity is, the brighter the red color becomes, that is, the magnitude of the blood flow velocity is related to the brightness of the red color. Further, the blood flow image to be displayed on the monitor 41 may be shown for example as an image in which when power value data is outputted from the dynamic blood flow detection circuit 35, the larger the blood flow intensity is, the brighter the red color becomes, that is, the magnitude of the blood flow intensity is related to the brightness of the red color.

Further, with the above described series of processing being performed by the ultrasound diagnostic apparatus 21, an operator or the like can visually recognize the blood flow velocity in the site where blood flow exists or dynamic blood flow while blood flow intensity is reduced, as a state in which the brightness of red color is gradually reduced as time elapses, in the blood flow image displayed on the monitor 41. Moreover, Fig. 4 is a graph to show temporal change of a luminance value in the portion where blood flow exists in the blood flow image displayed on the monitor 41, both when the above described series of processing has not be performed by the ultrasound diagnostic apparatus 21 and when the above described series of processing has been performed by the ultrasound diagnostic apparatus 21.

Moreover, in the present embodiment, the after image processing circuit 37 included in the Doppler processing circuit 24 of the ultrasound diagnostic apparatus 21 may be configured as an after image processing circuit 37A having approximately same effect as the after image processing circuit 37 as shown in Fig. 5.

The after image processing circuit 37A is configured to include a lookup table 37d, storage circuits 37f and 37g, and a comparison circuit 37h.

The storage circuit 37f made up of a frame memory and the like temporarily stores first luminance value data which is luminance value data in one frame and second luminance value data which is luminance value in the frame immediately preceding the one frame, out of the luminance value data outputted from the luminance value conversion circuit 36, thereafter outputting the first luminance value data to the comparison circuit 37h as well as outputting the second luminance data to the storage circuit 37g.

The storage circuit 37g, which is made up of a frame memory and the like, temporarily stores the second luminance value data outputted from the storage circuit 37f and thereafter outputs the second luminance value data to the lookup table 37d.

The lookup table 37d replaces the second luminance value data outputted from the storage circuit 37g with third luminance value data based on the comparative table included in the memory not shown, and thereafter outputs the third luminance value data to the comparison circuit 37h.

The comparison circuit 37h compares the first luminance value data outputted from the storage circuit 37f with the third luminance value data outputted based on the comparative table included in the lookup table 37d, and based on the comparison result, outputs one luminance value data which has a larger luminance value to the synthesis circuit 25.

The after image processing circuit 37A having a configuration as described above can achieve an approximately same effect as that of the after image processing circuit 37 as described above.

Further, although in the description of the present embodiment, the above described first, second, and third luminance values have been assumed to be all positive whole numbers not less than 0, for example 0 to 255, they are not limited thereto. For example, the luminance values may be values including color data having a positive or negative sign, for example, a whole number in the range of -127 to +127. In such a case, the after image processing circuit 37 included in the Doppler processing circuit 24 of the ultrasound diagnostic apparatus 21 is configured to be for example an after image processing circuit 37B as shown in Fig. 6.

The after image processing circuit 37B is configured to include a storage circuit 37a, a storage circuit 37b, a lookup table 37d, a comparison circuit 37p, multipliers 37q and 37r, a multiplication number setting circuit 37s for outputting a preset multiplication number, a switch 37t having two terminals t1 and t2, and a switch 37u having three terminals u1, u2 and u3.

The comparison circuit 37p compares the first luminance value based on the first luminance value data outputted from the storage circuit 37a with the second luminance value based on the second luminance value data outputted from the storage circuit 37b.

And when the first luminance value has a different sign from that of the second luminance value, the comparison circuit 37p outputs the first luminance value data to the switch 37u. Further, the comparison circuit 37p controls the switching of the switch 37u so as to change the terminal u1 into a conduction state so that the first luminance value data is outputted to the synthesis circuit 25.

Further, when the first luminance value and the second luminance value have the same sign, the comparison circuit 37p calculates the absolute value of the first luminance value and the absolute value of the second luminance value, and further performs the comparison based on the calculation result.

And, for example, when the absolute value of the first luminance value is larger than that of the second luminance value, the comparison circuit 37p outputs the first luminance value data to the switch 37u. Further, the comparison circuit 37p controls the switching of the switch 37u so as to change the terminal u2 into a conduction state so that the first luminance value data is outputted to the synthesis circuit 25.

Further, for example, when the absolute value of the first luminance value is not larger than that of the second luminance value, the comparison circuit 37p outputs the absolute value of the second luminance value to the multiplier 37q to cause a processing, which will be described below, to be performed at the multiplier 37q, the lookup table 37d and the multiplier 37r, and controls the switching of the switch 37u so as to change the terminal u3 into a conduction state so that the luminance value data obtained by the aforementioned processing is outputted to the synthesis circuit 25.

Further, when the second luminance value is not less than 0, the comparison circuit 37p controls the switching of the switch 37t so as to turn the terminal t1 into a conduction state. On the other hand, when the second luminance value is less than 0, the comparison circuit 37p controls the switching of the switch 37t so as to change the terminal t2 into a conduction state.

The multiplication number setting circuit 37s outputs, for example, 2 as the first multiplication number to the multiplier 37q; for example, 1/2 as the second multiplication number to the terminal t1 of the switch 37t; and for example, -1/2 as the third multiplication number to the terminal t2 of the switch 37, based on the preset multiplication number.

The absolute value of the second luminance value outputted from the comparison circuit 37p is doubled by being subjected to processing based on the first multiplication number outputted from the multiplication number setting circuit 37s at the multiplier 37q; and thereafter is outputted to the lookup table 37d.

Then, the absolute value of the second luminance value, which has been doubled and outputted from the multiplier 37q, is replaced with a third luminance value based on the comparative table included in the lookup table 37d, and thereafter is outputted to the multiplier 37r.

For example, when the second luminance value is not less than 0, the third luminance value outputted from the lookup table 37d is multiplied by 1/2 by being subject to processing based on the second multiplication number outputted from the multiplication number setting circuit 37s at the multiplier 37r; and thereafter is outputted to the synthesis circuit 25 via the terminal u3 of the switch 37u. On the other hand, when the second luminance value is less than 0, the third luminance value outputted from the lookup table 37d is multiplied by -1/2 by being subject to processing based on the third multiplication number outputted from the multiplication number setting circuit 37s at the multiplier 37r; and thereafter is outputted to the synthesis circuit 25 via the terminal u3 of the switch 37u.

The after image processing circuit 37B configured as described above can achieve approximately same effect as that of the after image processing circuit 37 described above using the same lookup table 37d as that of the after image processing circuit 37 even when a value including color data having a positive or negative sign is inputted.

The ultrasound diagnostic apparatus 21 of the present embodiment performs the processing based on the comparative table included in the lookup table 37d when showing dynamic blood flow during the decrease of blood flow velocity in the blood flow image displayed on the monitor 41. Therefore, the ultrasound diagnostic apparatus 21 of the present embodiment does not need to perform the processing to calculate the luminance value for each frame by arithmetic operation using a relatively complex function, for example by taking the 0.6th power of the inputted luminance value, as conventionally practiced when showing dynamic blood flow during the decrease of blood flow velocity. Since it is possible to preinstall calculation results using a complex function as described above into the comparative table included in the lookup table 37d, the ultrasound diagnostic apparatus 21 of the present embodiment can show dynamic blood flow during the decrease of blood flow velocity by a simpler configuration than ever without performing the processing which is conventionally performed and requires large load, for example performing the aforementioned calculation for each frame in the calculation circuit for calculating luminance values. As a result, the ultrasound diagnostic apparatus 21 of the present embodiment can show dynamic blood flow in the blood flow image displayed in the monitor 41 without decreasing the frame rate.

Further, the ultrasound diagnostic apparatus 21 of the present embodiment can show dynamic blood flow in a state in which the luminance value is gradually reduced as time elapses, by performing processing based on the comparative table included in the lookup table 37d when showing dynamic blood flow during the decrease of blood flow velocity, in the blood flow image displayed on the monitor 41. Therefore, the ultrasound diagnostic apparatus 21 of the present embodiment can show dynamic blood flow more accurately than ever.

The present invention will not be limited by the above described embodiment; and it goes without saying that various modifications and applications can be made.

## Claims

1. An ultrasound diagnostic apparatus (21), comprising:
ultrasound transmitting/receiving means (22) for transmitting an ultrasound pulse to generate ultrasound to be transmitted from ultrasound transducing means (12) to a test subject (101), and receiving an echo signal outputted from the ultrasound transducing means (12) based on the ultrasound reflected in the test subject (101);
dynamic blood flow detection means (35) for calculating a predetermined detection value based on dynamic blood flow in the test subject (101) based on the echo signal; and
luminance value conversion means (36) for converting the predetermined detection value into a luminance value;
luminance value replacement means (37d) for retaining luminance value replacement information for converting one luminance value to another luminance value; and
comparison means (37c) for comparing a first luminance value in one frame with a second luminance value in the frame immediately preceding the one frame, out of luminance values outputted from the luminance value conversion means (36), wherein the comparison means (37c) is adapted to output either the first luminance value or a third luminance value, which is not larger than the absolute value of the second luminance value, based on the comparison result and the luminance value replacement information,
**characterized in that** the comparison means is adapted to output the first luminance value when obtaining a comparison result that the first luminance value is larger than the second luminance value, and to replace the second luminance value with the third luminance value based on the luminance value replacement information, thereby outputting the third luminance value when obtaining a comparison result that the first luminance value is not larger than the second luminance value.

2. An ultrasound diagnostic apparatus (21), comprising:
ultrasound transmitting/receiving means (22) for transmitting an ultrasound pulse to generate ultrasound to be transmitted from ultrasound transducing means (12) to a test subject (101), and receiving an echo signal outputted from the ultrasound transducing means (12) based on the ultrasound reflected in the test subject (101);
dynamic blood flow detection means (35) for calculating a predetermined detection value based on dynamic blood flow in the test subject (101) based on the echo signal; and
luminance value conversion means (36) for converting the predetermined detection value into a luminance value;
luminance value replacement means (37d) for retaining luminance value replacement information for converting one luminance value to another luminance value; and
comparison means (37c) for comparing a first luminance value in one frame with a second luminance value in the frame immediately preceding the one frame, out of luminance values outputted from the luminance value conversion means (36),
**characterized in that** the comparison means (37c) is adapted to output either the first luminance value or a third luminance value, which is not larger than the absolute value of the second luminance value, based on the comparison result and the luminance value replacement information" wherein the comparison means (37c) is adapted to output the first luminance value when obtaining a comparison result that the first luminance value has a different sign from that of the second luminance value, or that the first luminance value has the same sign as that of the second luminance value and the absolute value of the first luminance value is larger than that of the second luminance value; and to replace the second luminance value with the third luminance value based on the luminance value replacement information and output the third luminance value, when obtaining a comparison result that the first luminance value has the same sign as that of the second luminance and the absolute value of the first luminance value is not larger than that of the second luminance value.

3. The ultrasound diagnostic apparatus according to one of claims 1-2, wherein the comparison means (37c) is adapted to increase the attenuation of the third luminance value as the second luminance value increases when replacing the second luminance value with the third luminance value based on the luminance value replacement information.

4. The ultrasound diagnostic apparatus of any preceding claim, further comprising:
a lookup table including a comparative table for converting the second luminance value into a third luminance value which is not larger than the absolute value of the second luminance value, wherein
the comparison means (37c) outputs either the first luminance value or the third luminance value based on the comparison result and the comparative table.

5. The ultrasound diagnostic apparatus of claim 4, wherein the comparative table retained in the lookup table is configured to be a rewritable data file.

6. The ultrasound diagnostic apparatus of claim 5, wherein the data file is made up of a plurality of data files.

## Patentansprüche

1. Ultraschall-Diagnosegerät (21) mit:
einer Ultraschall-Übertragungs/Empfangseinrichtung (22) zum Übertragen eines Ultraschallimpulses, um Ultraschall zu erzeugen, der von einer Ultraschall-Wandlereinrichtung (12) an ein Testsubjekt (101) zu übertragen ist, und zum Empfangen eines von der Ultraschall-Wandlereinrichtung (12) ausgegebenen Echosignals auf Basis des in dem Testsubjekt (101) reflektierten Ultraschalls;
einer dynamischen Blutströmungs-Detektionseinrichtung (35) zum Berechnen eines vorbestimmten Detektionswerts basierend auf einer dynamischen Blutströmung in dem Testsubjekt (101) auf Basis des Echosignals; und
einer Luminanzwert-Umwandlungseinrichtung (36) zum Umwandeln des vorbestimmten Detektionswerts in einen Luminanzwert;
einer Luminanzwert-Austauscheinrichtung (37d) zum Halten von Luminanzwert-Austauschinformation für eine Umwandlung eines Luminanzwerts in einen anderen Luminanzwert; und
einer Vergleichseinrichtung (37c) zum Vergleichen eines ersten Luminanzwerts in einem Frame mit einem zweiten Luminanzwert in dem dem einen Frame unmittelbar vorausgehenden Frame aus von der Luminanzwert-Umwandlungseinrichtung (36) ausgegebenen Luminanzwerten, wobei die Vergleichseinrichtung (37c) entweder den ersten Luminanzwert oder einen dritten Luminanzwert auszugeben vermag, der nicht größer ist als der Absolutwert des zweiten Luminanzwerts, basierend auf dem Vergleichsergebnis und der Luminanzwert-Austauschinformation,
**dadurch gekennzeichnet, dass** die Vergleichseinrichtung den ersten Luminanzwert auszugeben vermag, wenn sie ein Vergleichsergebnis erhält, dass der erste Luminanzwert größer als der zweite Luminanzwert ist, und den zweiten Luminanzwert gegen den dritten Luminanzwert auf Basis der Luminanzwert-Austauschinformation auszutauschen vermag, wodurch sie den dritten Luminanzwert ausgibt, wenn sie ein Vergleichsergebnis erhält, dass der erste Luminanzwert nicht größer als der zweite Luminanzwert ist.

2. Ultraschall-Diagnosegerät (21) mit:
einer Ultraschall-Übertragungs/Empfangseinrichtung (22) zum Übertragen eines Ultraschallimpulses, um Ultraschall zu erzeugen, der von einer Ultraschall-Wandlereinrichtung (12) an ein Testsubjekt (101) zu übertragen ist, und zum Empfangen eines von der Ultraschall-Wandlereinrichtung (12) ausgegebenen Echosignals auf Basis des in dem Testsubjekt (101) reflektierten Ultraschalls;
einer dynamischen Blutströmungs-Detektionseinrichtung (35) zum Berechnen eines vorbestimmten Detektionswerts basierend auf einer dynamischen Blutströmung in dem Testsubjekt (101) auf Basis des Echosignals; und
einer Luminanzwert-Umwandlungseinrichtung (36) zum Umwandeln des vorbestimmten Detektionswerts in einen Luminanzwert;
einer Luminanzwert-Austauscheinrichtung (37d) zum Halten von Luminanzwert-Austauschinformation für eine Umwandlung eines Luminanzwerts in einen anderen Luminanzwert; und
einer Vergleichseinrichtung (37c) zum Vergleichen eines ersten Luminanzwerts in einem Frame mit einem zweiten Luminanzwert in dem dem einen Frame unmittelbar vorausgehenden Frame aus von der Luminanzwert-Umwandlungseinrichtung (36) ausgegebenen Luminanzwerten,
**dadurch gekennzeichnet, dass** die Vergleichseinrichtung (37c) entweder den ersten Luminanzwert oder einen dritten Luminanzwert auszugeben vermag, der nicht größer ist als der Absolutwert des zweiten Luminanzwerts, basierend auf dem Vergleichsergebnis und der Luminanzwert-Austauschinformation, wobei die Vergleichseinrichtung (37c) den ersten Luminanzwert auszugeben vermag, wenn sie ein Vergleichsergebnis erhält, dass der erste Luminanzwert ein anderes Vorzeichen hat als der zweite Luminanzwert, oder dass der erste Luminanzwert das gleiche Vorzeichen hat wie der zweite Luminanzwert und der Absolutwert des ersten Luminanzwerts größer ist als der des zweiten Luminanzwerts; und dass sie den zweiten Luminanzwert gegen den dritten Luminanzwert auf Basis der Luminanzwert-Austauschinformation auszutauschen und den dritten Luminanzwert auszugeben vermag, wenn sie ein Vergleichsergebnis erhält, dass der erste Luminanzwert das gleiche Vorzeichen hat wie das der zweiten Luminanz, und der Absolutwert des ersten Luminanzwerts nicht größer ist als der des zweiten Luminanzwerts.

3. Ultraschall-Diagnosegerät nach einem der Ansprüche 1-2, wobei die Vergleichseinrichtung (37c) die Dämpfung des dritten Luminanzwerts bei zunehmendem zweiten Luminanzwert zu verstärken vermag, wenn der zweite Luminanzwert gegen den dritten Luminanzwert auf Basis der Luminanzwert-Austauschinformation ausgetauscht wird.

4. Ultraschall-Diagnosegerät nach einem der vorangehenden Ansprüche, ferner mit:
einer Lookup-Tabelle mit einer Vergleichstabelle zum Umwandeln des zweiten Luminanzwerts in einen dritten Luminanzwert, der nicht größer als der Absolutwert des zweiten Luminanzwerts ist, wobei
die Vergleichseinrichtung (37c) entweder den ersten Luminanzwert oder den dritten Luminanzwert auf Basis des Vergleichsergebnisses und der Vergleichstabelle ausgibt.

5. Ultraschall-Diagnosegerät nach Anspruch 4, wobei die in der Lookup-Tabelle enthaltene Vergleichstabelle als überschreibbare Datendatei konfiguriert ist.

6. Ultraschall-Diagnosegerät nach Anspruch 5, wobei die Datendatei aus mehreren Datendateien zusammengesetzt ist.

## Revendications

1. Appareil de diagnostic à ultrasons (21), comprenant :
un moyen d'émission/réception d'ultrasons (22) pour émettre une impulsion ultrasonore pour générer un ultrason devant être transmis d'un moyen de transduction d'ultrasons (12) jusqu'à un sujet de test (101), et recevoir un signal d'écho délivré en sortie du moyen de transduction d'ultrasons (12) sur la base de l'ultrason réfléchi dans le sujet de test (101) ;
un moyen de détection de flux sanguin dynamique (35) pour calculer une valeur de détection prédéterminée sur la base du flux sanguin dynamique dans le sujet de test (101) sur la base du signal d'écho ; et
un moyen de conversion de valeur de luminance (36) pour convertir la valeur de détection prédéterminée en une valeur de luminance ;
un moyen de remplacement de valeur de luminance (37d) pour conserver une information de remplacement de valeur de luminance pour convertir une valeur de luminance en une autre valeur de luminance ; et
un moyen de comparaison (37c) pour comparer une première valeur de luminance dans une trame avec une deuxième valeur de luminance dans la trame précédant immédiatement la trame, parmi des valeurs de luminance délivrées en sortie du moyen de conversion de valeur de luminance (36), dans lequel le moyen de comparaison (37c) est adapté à délivrer en sortie soit la première valeur de luminance, soit une troisième valeur de luminance, qui n'est pas plus grande que la valeur absolue de la deuxième valeur de luminance, sur la base du résultat de comparaison et de l'information de remplacement de valeur de luminance,
**caractérisé en ce que** le moyen de comparaison est adapté à délivrer en sortie la première valeur de luminance à l'obtention d'un résultat de comparaison dans lequel la première valeur de luminance est plus grande que la deuxième valeur de luminance, et à remplacer la deuxième valeur de luminance par la troisième valeur de luminance sur la base de l'information de remplacement de valeur de luminance, délivrant ainsi en sortie la troisième valeur de luminance à l'obtention d'un résultat de comparaison dans lequel la première valeur de luminance n'est pas plus grande que la deuxième valeur de luminance.

2. Appareil de diagnostic à ultrasons (21), comprenant :
un moyen d'émission/réception d'ultrasons (22) pour émettre une impulsion ultrasonore pour générer un ultrason devant être transmis d'un moyen de transduction d'ultrasons (12) jusqu'à un sujet de test (101), et recevoir un signal d'écho délivré en sortie du moyen de transduction d'ultrasons (12) sur la base de l'ultrason réfléchi dans le sujet de test (101) ;
un moyen de détection de flux sanguin dynamique (35) pour calculer une valeur de détection prédéterminée sur la base du flux sanguin dynamique dans le sujet de test (101) sur la base du signal d'écho ; et
un moyen de conversion de valeur de luminance (36) pour convertir la valeur de détection prédéterminée en une valeur de luminance ;
un moyen de remplacement de valeur de luminance (37d) pour conserver une information de remplacement de valeur de luminance pour convertir une valeur de luminance en une autre valeur de luminance ; et
un moyen de comparaison (37c) pour comparer une première valeur de luminance dans une trame avec une deuxième valeur de luminance dans la trame précédant immédiatement la trame, parmi des valeurs de luminance délivrées en sortie du moyen de conversion de valeur de luminance (36),
**caractérisé en ce que** le moyen de comparaison (37c) est adapté à délivrer en sortie soit la première valeur de luminance, soit une troisième valeur de luminance, qui n'est pas plus grande que la valeur absolue de la deuxième valeur de luminance, sur la base du résultat de comparaison et de l'information de remplacement de valeur de luminance, dans lequel le moyen de comparaison (37c) est adapté à délivrer en sortie la première valeur de luminance à l'obtention d'un résultat de comparaison dans lequel la première valeur de luminance a un signe différent de celui de la deuxième valeur de luminance, ou que la première valeur de luminance a le même signe que celui de la deuxième valeur de luminance et que la valeur absolue de la première valeur de luminance est plus grande que celle de la deuxième valeur de luminance ; et à remplacer la deuxième valeur de luminance par la troisième valeur de luminance sur la base de l'information de remplacement de valeur de luminance et délivrer en sortie la troisième valeur de luminance, à l'obtention d'un résultat de comparaison dans lequel la première valeur de luminance a le même signe que celui de la deuxième valeur de luminance et que la valeur absolue de la première valeur de luminance n'est pas plus grande que celle de la deuxième valeur de luminance.

3. Appareil de diagnostic à ultrasons selon l'une des revendications 1 à 2, dans lequel le moyen de comparaison (37c) est adapté à augmenter l'atténuation de la troisième valeur de luminance lorsque la deuxième valeur de luminance augmente lors du remplacement de la deuxième valeur de luminance par la troisième valeur de luminance sur la base de l'information de remplacement de valeur de luminance.

4. Appareil de diagnostic à ultrasons selon une quelconque revendication précédente, comprenant en outre :
une table de recherche incluant une table comparative pour convertir la deuxième valeur de luminance en une troisième valeur de luminance qui n'est pas plus grande que la valeur absolue de la deuxième valeur de luminance, dans lequel
le moyen de comparaison (37c) délivre en sortie soit la première valeur de luminance, soit la troisième valeur de luminance sur la base du résultat de comparaison et de la table comparative.

5. Appareil de diagnostic à ultrasons selon la revendication 4, dans lequel la table comparative conservée dans la table de recherche est configurée pour être un fichier de données réinscriptible.

6. Appareil de diagnostic à ultrasons selon la revendication 5, dans lequel le fichier de données est constitué d'une pluralité de fichiers de données.
